(19) Europäisches Patentamt
European Patent Office
Office européen des brevets

(11) Publication number: **0 155 825**
**A2**

# (12) EUROPEAN PATENT APPLICATION

(21) Application number: 85301793.7

(22) Date of filing: 14.03.85

(51) Int. Cl.[4]: C 07 C 87/30
C 07 C 85/00, C 12 N 9/00

(30) Priority: 21.03.84 JP 54066/84

(43) Date of publication of application:
25.09.85 Bulletin 85/39

(84) Designated Contracting States:
CH DE FR GB LI SE

(72) Inventor: Kuroiwa, Katsumasa
1-44, Aza Shimokitaimae
Arai Asakacho Koriyama-shi(JP)

(72) Inventor: Katayama, Katsuhiro
198 Aza Ohara Kubota
Fukuyamacho Koriyama-shi(JP)

(72) Inventor: Takabayashi, Katsuyuki
37-14, Aza Shigoda
Mougicho Koriyama-shi(JP)

(72) Inventor: Nagasawa, Takeshi
19-10 Ryoke-7-chome
Urawa-shi(JP)

(74) Representative: Grundy, Derek George Ritchie et al,
CARPMAELS & RANSFORD 43, Bloomsbury Square
London WC1A 2RA(GB)

## (54) Novel choline derivatives.

(57) This invention relates to novel choline derivatives 3,4-dihydroxybenzoylcholine halides of the formula

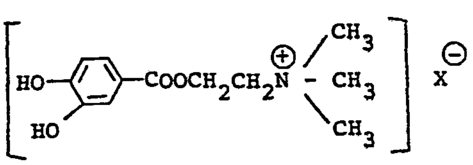

wherein X represents a halogen atom. The novel choline derivatives are of great use as a substrate in the assay of enzymatic activity of cholinesterase.

FIG. 2

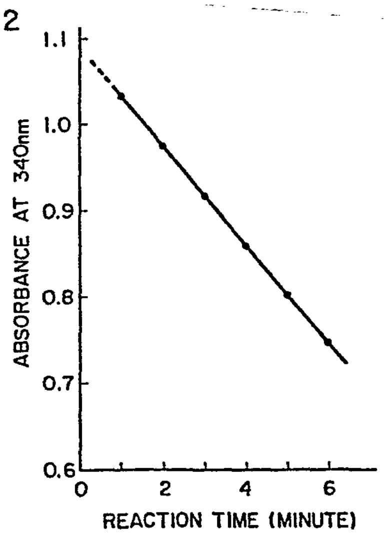


EP 0 155 825 A2

0155825

# NOVEL CHOLINE DERIVATIVES

1 This invention relates to novel choline derivatives 3,4-dihydroxybenzoylcholine halides represented by the general formula

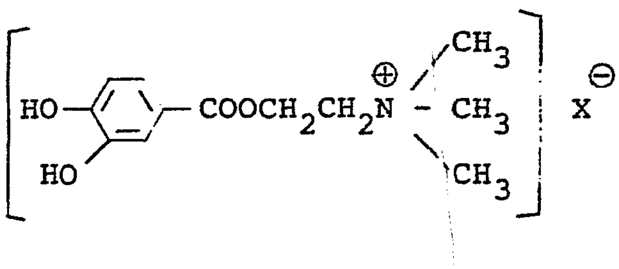

(I)

wherein X represents a halogen atom.

As shown later in Example 1, the above compounds are synthesized from 3,4-dihydroxybenzoic acid as starting material by first protecting the hydroxyl groups at positions 3 and 4 with carbobenzoxyl groups by reacting with carbobenzoxy chloride, then converting the protected compound into an acyl chloride in a usual manner of chemical synthesis by using thionyl chloride, phosphorus pontachloride, or the like, condensing the resulting acyl chloride with N,N-dimethylaminoethanol by dehydrochlorination, then removing the protective groups by hydrogenation to obtain N,N-dimethylaminoethyl 3,4-dihydroxybenzoate which is then quaternized with a methyl halide to yield 3,4-dihydroxybenzoylcholine halide, a novel compound of this invention represented by the general formula (I).

The novel compounds of this invention are very useful as the substrate in the assay of enzymatic activity

of cholinesterase (enzyme code number of 3.1.1.8; referred to briefly as Ch-E). A detailed description about the methods of said assay is given hereunder:

There have, heretofore, been reported various methods for assaying enzymatic activity of Ch-E in blood by using synthetic substrates and some of the methods are currently being used in the routine clinical examination. These methods, however, have both merits and demerits and, in addition, have own problems leading to incorrect results of assay. As examples of the methods of assay, mention may be made of gas analysis method, pH meter method, pH indicator colorimetric method, thiocholine color reaction method, enzymatic method, and UV method.

In the gas analysis method [R. Ammon, Pflügers Arch. Ges. Physiol., 233, 487 (1933)], acetylcholine is employed as the synthetic substrate and measurement is made of the carbon dioxide generated from sodium hydrogencarbonate by the reaction with the acetic acid which is formed by the enzymatic action of Ch-E. The disadvantages of this method are complicated procedure and inability to treat a large number of specimens.

The pH meter method [H. O. Michel, J. Lab. & Clin. Med., 34, 1564 (1949)] is a method in which the acetic acid formed by the enzymatic activity of Ch-E similarly to the gas analysis method is determimined by the change in pH measured by means of a pH meter. The problems of this method resides in accuracy of the pH meter and inability to treat a large number of specimens.

The pH indicator colorimetric method is different from the pH meter method in that the pH change due to the formation of acetic acid caused by the activity of Ch-E is determined by measuring the molar absorbance of the indicator. As the indicator, there are used Phenol Red [H. Takahashi and S. Shibata, Medical Science and Biology, 20, 96 (1951)], Bromothymol Blue [H. G. Biggs et al., Amer. J. Clin. Path., 30, 181 (1958)], and m-nitrophenol [M. Sasaki, Clinical Pathology, 12, 555 (1964)]. It is pointed out that although this method is simple in procedure and is able to treat a large number of specimens, it has disadvantages in that the reaction time is so long that pH changes during the reaction, and the reproducibility of the measurement is not so good in higher and lower pH regions.

In the above methods which employ acetylcholine as substrate, the substrate itself presents a problem, because acetylcholine is susceptible to non-enzymatic hydrolysis and is unsatisfactory in substrate specificity.

The thiocholine method [P. Garry, J. Clin. Chem., 11 (2), 91 (1965)] employs as substrate acetylthiocholine, propylthiocholine, or butylthiocholine which liberates thiocholine by the enzymatic action of Ch-E. The liberated thiocholine reacts with 5,5'-dithiobis-2-nitrobenzoic acid (DTNB) to develop a yellow color, the absorbance of which is colorimetrically measured. This method has advantages of excellent reactivity, high sensitivity, simple procedure, capability of

treating a large number of specimens, and adaptability to the method of initial rate of reaction. However, the colorimetry of yellow color is strongly affected by bilirubin contained in the sample (for example, serum and plasma), and also cannot escape the effect of SH-containing compounds such as glutathione. Other problems of the method include the instability of the substrate itself.

The enzymatic method utilizes as substrate benzoylcholine [H. Okabe et al., Clinical Pathology, 25, 751 (1977)] or o-toluoylcholine [Japanese Patent Application "Kokai" (Laid-open) No. 138,533/79], which liberate choline by the enzymatic action of Ch-E. The liberated choline is then converted into betain by the action of choline-oxidase. The hydrogen peroxide formed in said conversion is allowed in the presence of peroxidase to effect the oxidative condensation between 4-aminoantipyrine with phenol or the like to develop a color. Because of the coloration in red, this method is free from the interference from bilirubin in the sample (e.g. serum or plasma). Another advantage of the method is that a large number of specimens can be treated. However, since the phenol and 4-aminoantipyrine used as reagents in the color reaction system have a competitive inhibitory effect upon Ch-E, the amounts of these reagents in the color reaction system are limited to so narrow a range that full development of color is difficult to attain.

In general the method of assay by way of hydrogen peroxide is difficult to avoid the influence of a reducing substance such as bilirubine or ascorbic acid in the sample (e.g. serum or plasma), and is also subject to the influence of choline formed by the decomposition of phospholipids. When benzoylcholine is used as substrate, the non-enzymatic hydrolysis becomes one of the problems.

The UV method includes two types of procedures. The substrate employed is benzoylcholine [W. Kalow and K. Genet, Canad. J. Biochem. & Physiol., 35, 339 (1957)] in the one and hydroxybenzoylcholine in the other. In the former procedure, the consumption of substrate due to hydrolysis caused by the enzymatic activity of Ch-E is traced through the change in absorbance at 240 nm. The principle of this type of assay which is based on the direct measurement of decreasing substrate is simple and straightforward. However, an important problem of this method is a narrow range of linearity owing to the limited concentration of the substrate in the reactant mixture, because the measurement which is performed at a short wavelength is susceptible to the interference from the constituents of serum or plasma. Other problems are such that the benzoylcholine gives rise to substrate inhibition and that because of the susceptibility of benzoylcholine to non-enzymatic hydrolysis, the reaction is not carried out at the optimum pH of Ch-E. In the latter type procedure [Japanese Patent Application "Kokai"

(Laid-open) No. 110,198/82 and No. 152,500/83] p-hydroxybenzoylcholine is used as substrate and subjected to the enzymatic activity of Ch-E in the presence of coenzyme NADPH; the p-hydroxybenzoic acid which is formed is traced by measuring the decrease in absorbance at wavelength 340 nm, which is caused by the oxidation of coenzyme NADPH to NADP. This procedure is an excellent method for assaying the activity of Ch-E, because it allows the reaction to proceed at the optimum pH; it is free from the disadvantages of the method of hydrogen peroxide-color reaction, such as the influence of reducing substances including bilirubin and ascorbic acid, and the interference exerted by the choline formed from decomposition of phospholipids; it is also free from the disadvantages of thiocholine method; and it is suitable for use in an automatic analyzer capable of treating a large numbers of specimens. However, this procedure employs coenzyme NADPH which is an expensive and labile reagent which is difficult to maintain and control at a constant level of quality and also requires such enzymes as p-hydroxybenzoic acid hydroxylase and protocatechuic acid-3,4-dioxygenase which complicate the procedure compared with the former procedure and, hence, tend to increase error of measurement.

As described above, conventional methods of assaying enzymatic activity of Ch-E or the synthetic substrates used in said methods present various problems. The present inventors carried out an extensive study to

solve the problems and, as a result, found that the novel 3,4-dihydroxybenzoylcholine halide is a very useful substrate in the assay of enzymatic activity of Ch-E. This finding has led to the accomplishment of the present invention relating to the method of conveniently and precisely assaying the enzymatic activity of Ch-E in blood. The utility of the present substrate is described below.

Fig. 1 represents UV spectra of 3,4-dihydroxybenzoylcholine iodide (hereinafter referred to briefly as DHBCI) and 3,4-dihydroxybenzoic acid. When DHBCI is hydrolyzed by the action of Ch-E, there are formed choline and 3,4-dihydroxybenzoic acid. The choline shows no UV absorption above a wavelength of 340 nm, whereas 3,4-dihydroxybenzoic acid shows almost no UV absorption below a wavelength of 340 nm. Therefore, when DHBCI, a novel compound according to the present invention, is used as substrate in the assay of enzymatic activity of Ch-E and the reaction is traced through the absorbance at a wavelength of 340-360 nm, it is possible to follow precisely the decrease in substrate DHBCI. In the previously mentioned method of W. Kalow, since the measurement is done at a shorter wavelength of 240 nm, the initial absorption is interfered with by the blood constituents, whereas at a wavelength of 340-360 nm, the interference is very little and the conditions of reaction can be set up more easily. The substrate DHBCI is very stable against the non-enzymatic hydrolysis

and shows substantially no hydrolysis in a 1/15 molar phosphate buffer (pH 8.20) at 37°C for 90 minutes, indicating that the non-enzymatic hydrolysis during the measurement can be neglected. The Km value is not much different from that of benzoylcholine and is $2.6 \times 10^{-5}$ M in 50 mM TRIS-maleate buffer (pH 8.20). This value of the substrate DHBCI is sufficiently small so that the reaction is allowed to proceed in the reaction system in the presence of the substrate of a sufficient concentration. As a consequence, the range of linear relationship with time becomes sufficiently large to assay the activity of Ch-E in blood.

The usefulness of the novel compound of this invention is described below with reference to Referential Examples.

REFERENTIAL EXAMPLE 1

The time course was as shown in Fig. 2. The assay was performed by adjusting the substrate concentration to 0.25 mM with a 50 mM TRIS-maleate buffer (pH 8.20), preheating for about 5 minutes 2.0 mℓ of the adjusted solution to 37°C, admixing the preheated solution with 200 μℓ of a serum, and immediately measuring the absorbance decrease at 340 nm of the mixture at 37°C. The serum used was consera I (Nissui Seiyaku Co.). The activity of Ch-E was calculated from the following equation:

$$IU/\ell = \frac{\Delta O.D.^{*} \times \begin{pmatrix}\text{Volume of}\\ \text{reaction}\\ \text{mixture}\end{pmatrix} \times 1000}{\begin{pmatrix}\text{Molecular}\\ \text{extinction}\\ \text{coeffi-}\\ \text{cient}^{**}\end{pmatrix} \times \begin{pmatrix}\text{Volume}\\ \text{of serum}\end{pmatrix}}$$

* ΔO.D.: Absorbance change per minute at a wavelength of 340 nm.

** Molar absorbance at 340 nm: 2960

The calculated value of the serum used was 216 units (IU/ℓ). As shown in Fig. 2, the absorbance of Ch-E having an activity of 216 units (IU/ℓ) was in a linear relationship with time for a period of 6 minutes, indicating that the present method of assay is adaptable to automatic analyzer.

REFERENTIAL EXAMPLE 2

The relationships between the dilution ratio of serum and the enzymatic activity with respect to a few sera were as shown in Fig. 3. The conditions of assay were as follows:

a. Substrate: DHBCI

b. Buffer: 50 mM TRIS-maleate (pH 8.20)

c. Sera:

| | | |
|---|---|---|
| (1) | Consera I | 100 μℓ |
| (2) | Consera I | 200 μℓ |
| (3) | Precipath E (Boehringer Mannheim Co.) | 100 μℓ |

Each serum was diluted with physiological

saline. The substrate concentraion was adjusted to 0.25 mM with a buffer solution. The measurement was carried out similarly to the measurement in Referential Example 1 by adding each serum or diluted serum in an amount of 100 μℓ [(1) or (3)] or 200 μℓ [(2)].

As shown in Fig. 3, each of the three sera showed satisfactory linear relationship between the dilution ratio of serum and the enzymatic activity.

From the results of Referential Examples 1 and 2, it has become apparent that DHBCI allows the assay of Ch-E activity in the blood of various potencies ranging from low potency specimen to high potency one to carry out by the simple procedure based on a simple reaction mechanism. The results proved the usefulness of the present 3,4-dihydroxybenzoylcholine as a substrate in the assay of Ch-E activity in blood.

The synthesis of novel 3,4-dihydroxybenzoylcholine iodide according to this invention is described in the following Example. The iodide can be converted into other halides in a manner common to the synthetic chemistry.

EXAMPLE 1

Into 71 mℓ of 2.73 N sodium hydroxide solution, was dissolved 10 g of 3,4-dihydroxybenzoic acid. To the solution cooled at 0°-5°C in ice and vigorously stirred, was added dropwise 22 mℓ of carbobenzoxy chloride together with 2.73 N sodium hydroxide solution

to maintain pH at 9-10. After about one hour, pH became constant. The mixture was then stirred at room temperature for 3 hours to allow the reaction to proceed. The mixture was then adjusted to pH 2 with cold 5 N hydrochloric acid and extracted successively with 200 mℓ and 100 mℓ of ethyl acetate. The combined ethyl acetate phase was washed with a sodium chloride solution, dried over anhydrous magnesium sulfate, and the solvent was evaporated under reduced pressure, leaving behind 25 g of an oily substance. The oily substance was purified by silica gel chromatography to yield 9.2 g of O,O'-dicarbobenzoxy-3,4-dihydroxybenzoic acid. To a suspension of 4 g of this substance in 50 mℓ of ether, under moistrue-proof conditions, was added 2 g of powdered phosphorus pentachloride. The mixture, while being stirred, was allowed to react at room temperature for 5 hours. After completion of the reaction, the solvent was removed by distillation under reduced pressure to yield 4.5 g of an oily substance. The oily substance was dissolved in 20 mℓ of benzene and the resulting solution was added dropwise to a solution of 2 mℓ of dimethylaminoethanol in 30 mℓ of benzene, while being stirred and cooled at 5°-10°C in ice. After the addition, the resulting mixture was allowed to react overnight at room temperature, while being stirred. The reaction mixture was diluted with 50 mℓ of benzene, washed with water, then with saturated aqueous sodium chloride solution. The benzene phase was dried over

anhydrous magnesium sulfate, and the solvent was evaporated under reduced pressure to yield 4.9 g of an oily substance was dissolved in 260 mℓ of ethanol and catalytically reduced with 2 g of palladium black for 5 hours. After completion of the reduction, the reaction mixture was filtered from the catalyst, and the ethanol was evaporated under reduced pressure to yield 3 g of an oily substance. The oily substance was dissolved in 90 mℓ of acetone and the solution was admixed with a solution of 2 g of methyl iodide in ethyl acetate. The mixture was allowed to stand overnight at room temperature to precipitate crystals. The crystals were collected by filtration, washed thoroughly with acetone, and dried overnight over phosphorus pentoxide under reduced pressure to yield 2.5 g of 3,4-dihydroxybenzoylcholine iodide, a novel compound of this invention; melting point 206°-209°C. Upon silica gel thin layer chromatography (n-butanol : acetic acid : water = 4 : 1 : 2), said crystalline compound gave a single spot ($R_f$ = 0.31).

Elementary analysis:

| | C% | H% | N% |
|---|---|---|---|
| Found | 39.34 | 5.07 | 3.90 |
| Calculated for $C_{12}H_{18}NO_4I$ | | | |
| (molecular weight: 367.166) | 39.25 | 4.94 | 3.81 |

The infrared absorption spectrum was as shown in Fig. 4

## BRIEF DESCRIPTION OF THE DRAWINGS

Fig. 1 represents UV spectra of 3,4-dihydroxybenzoylcholine iodide (a) and 3,4-dihydroxybenzoic acid (b) in 1/15 M phosphate buffer (pH 8.20).

Fig. 2 and 3, respectively, represent the relationship between the Ch-E activity in serum and the reaction time with dihydroxybenzoylcholine iodide as substrate and the relationship between the dilution ratio of serum and the Ch-E activity with the said compound as substrate.

Fig. 4 represents the IR absorption spectrum of 3,4-dihydroxybenzoylcholine iodide.

WHAT IS CLAIMED IS:

1. 3,4-Dihydroxybenzoylcholine halides represented by the general formula

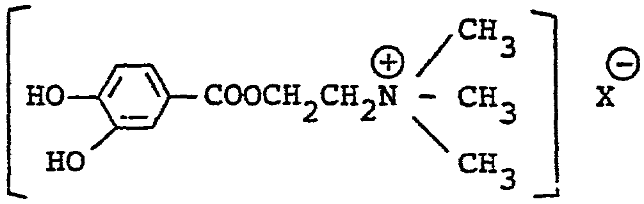

(I)

wherein X represents a halogen atom.

2. A method of preparing compounds of the general formula I, as defined in Claim 1, which comprises the steps of reacting 3,4-dihydroxybenzoic acid with an agent which provides protecting groups for the hydroxyl groups at positions 3 and 4 of the benzene ring; reacting the protected compound with a suitable chlorinating agent to form the acyl chloride; condensing the acyl chloride with N,N-dimethylaminoethanol; hydrogenating the resultant compound to remove the protecting groups and quarternizing the product so formed with a methyl halide.

3. Use of a compound of the general formula I, as defined in Claim 1, as a substrate in the assay of the enzymatic activity of cholinesterase.

4. A method of assaying the enzymatic activity of cholinesterase in a given sample which comprises mixing the sample with a predetermined quantity of a compound of the general formula I, as defined in Claim 1, and monitoring the UV absorbance of the mixture at a selected wavelength for a selected period of time.

5. A method according to Claim 4 in which the sample is a blood sample.

6. A method according to Claim 4 or 5 in which the selected wavelength is in the range from 340-360 nm.

7. A method according to Claim 6 in which the selected wavelength is 340 nm.

FIG. 1

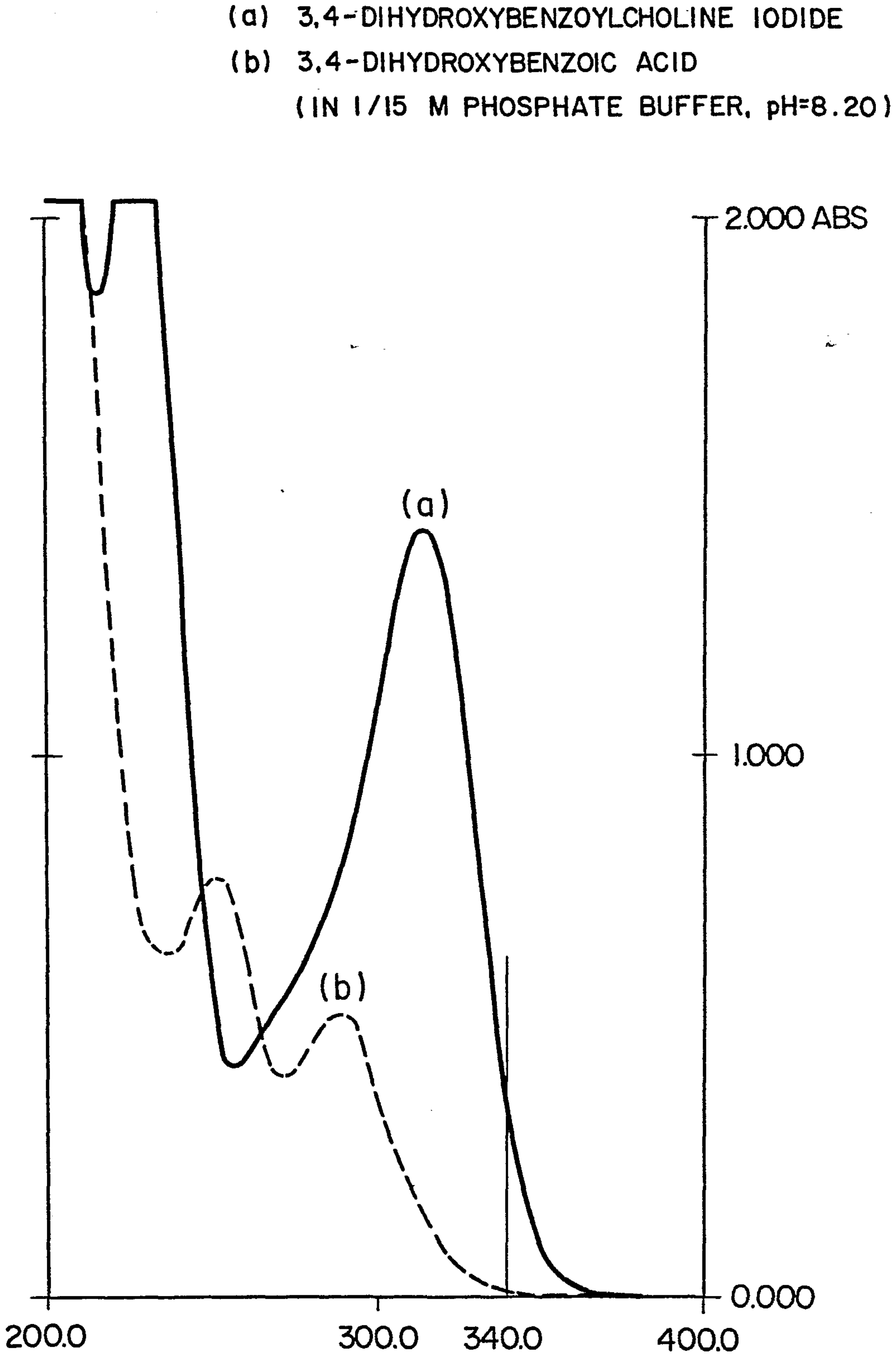
(a) 3,4-DIHYDROXYBENZOYLCHOLINE IODIDE
(b) 3,4-DIHYDROXYBENZOIC ACID
(IN 1/15 M PHOSPHATE BUFFER, pH=8.20)
2.000 ABS
1.000
0.000
(a)
(b)
200.0
300.0
340.0
400.0

FIG. 2

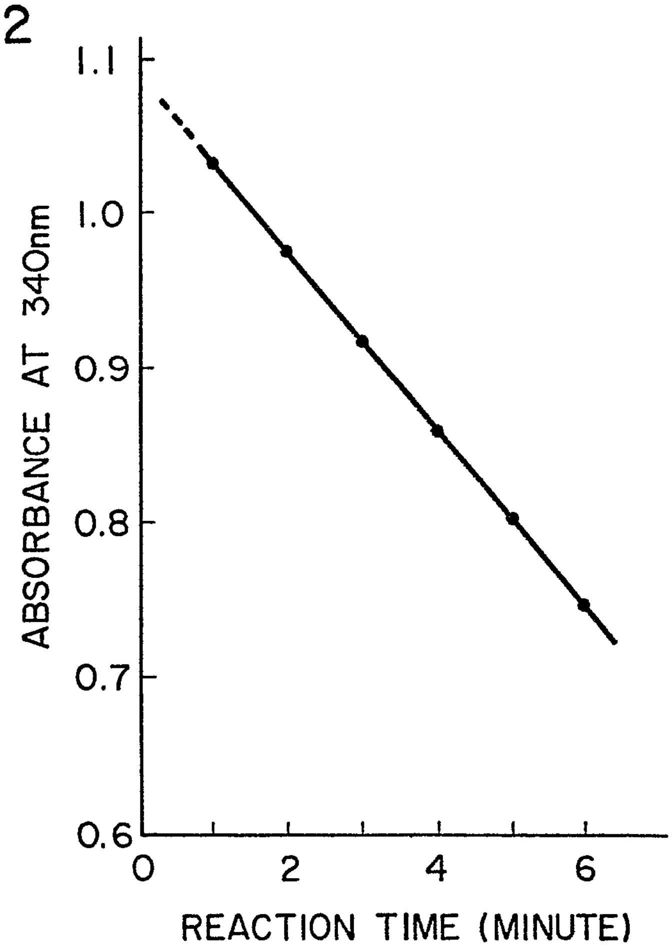
ABSORBANCE AT 340nm
1.1
1.0
0.9
0.8
0.7
0.6
0
2
4
6
REACTION TIME (MINUTE)

FIG. 4

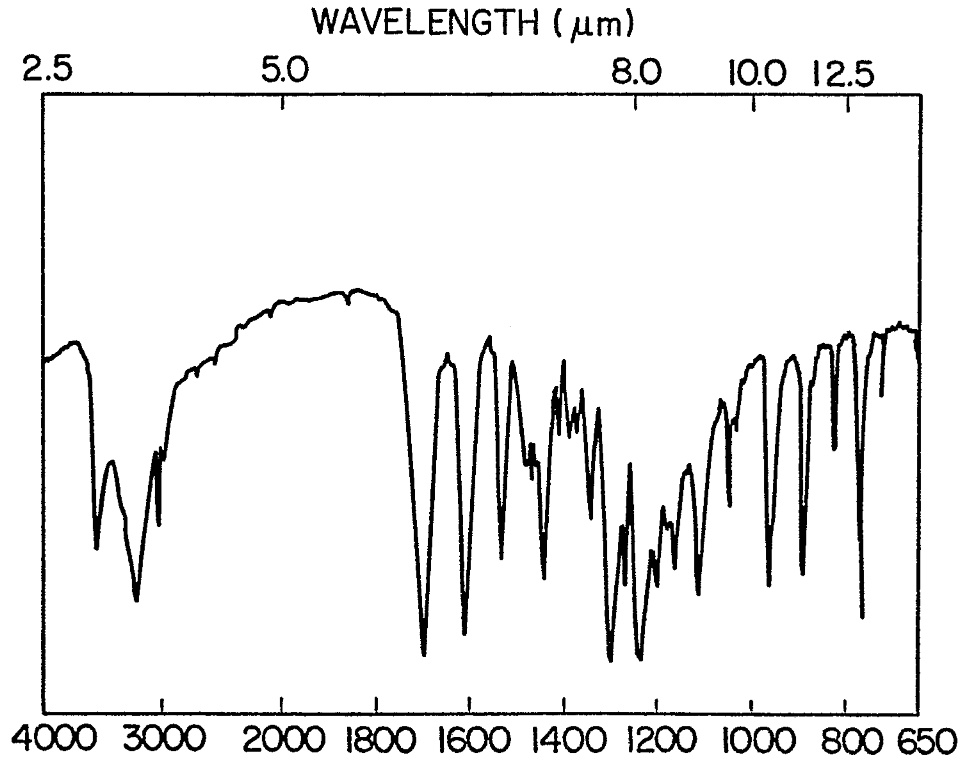
WAVELENGTH (μm)
2.5
5.0
8.0
10.0
12.5
4000
3000
2000
1800
1600
1400
1200
1000
800
650

FIG. 3

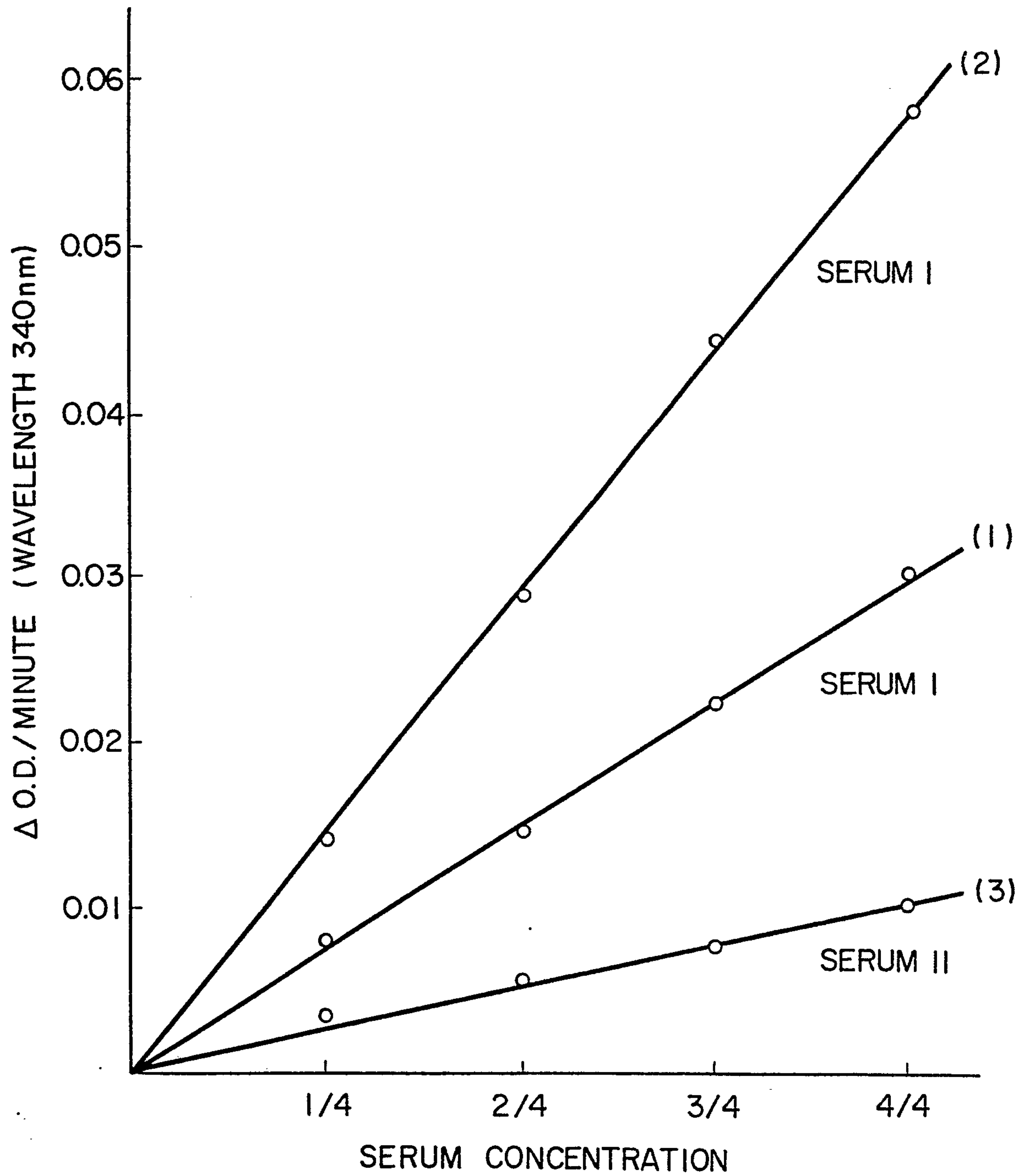

Δ O.D./MINUTE (WAVELENGTH 340nm)
0.06
0.05
0.04
0.03
0.02
0.01
(2)
SERUM I
(1)
SERUM I
(3)
SERUM II
1/4
2/4
3/4
4/4
SERUM CONCENTRATION